Europäisches Patentamt

European Patent Office

Office européen des brevets

(11) Publication number: **0 255 875**
**B1**

# EUROPEAN PATENT SPECIFICATION

(45) Date of publication of patent specification: **27.12.90**

(51) Int. Cl.⁵: **A 01 N 35/02**

(21) Application number: **87109830.7**

(22) Date of filing: **08.07.87**

(54) **Glutaraldehyde-based sterilising composition of antibacterial and antimycotic activity in an aqueous vehicle.**

(30) Priority: **08.08.86 IT 2145086**

(43) Date of publication of application:
**17.02.88 Bulletin 88/07**

(45) Publication of the grant of the patent:
**27.12.90 Bulletin 90/52**

(84) Designated Contracting States:
**AT BE CH DE ES FR GB GR IT LI LU NL SE**

(56) References cited:
**FR-A-2 196 816**
**US-A-3 912 450**
**US-A-3 917 850**
**US-A-4 103 001**
**US-A-4 208 404**
**US-A-4 469 614**

(73) Proprietor: **GERMO S.P.A.**
**via Giotto 19/21**
**I-20032 Cormano (MI) (IT)**

(72) Inventor: **Magni, Luigi Fabio**
**P.zza Giulio Cesare 7**
**I - Milano (IT)**

(74) Representative: **Gervasi, Gemma, Dr.**
**Studio Brevetti e Marchi NOTARBARTOLO &**
**GERVASI 33, Viale Bianca Maria**
**I-20122 Milano (IT)**

The file contains technical information submitted after the application was filed and not included in this specification

Courier Press, Leamington Spa, England.

**Description**

This invention relates to an aqueous chemical composition of considerable sporicidal, virucidal, fungicidal and bactericidal power suitable for use as a disinfectant sterilising at ambient temperature and possessing considerable stability with time. The disinfecting and sterilisation of equipment and devices used in the diagnostic and therapeutic field such as equipment for dental and hemodialysis use, and the disinfecting of treatment environments and the clothes of the patient or assisting personnel, require the use of products which as far as possible satisfy the following requirements:

    — wide range of action

    — short contact time

    — low toxicity

    — moderate cost

    — suitability for use at ambient temperature

    — absence of corrosion of the treated objects, especially if metallic.

Considerable sporicidal power is also of fundamental importance in obtaining reliable and total sterilisation.

A composition which has been widely used in recent years is one based on glutaraldehyde in aqueous solution.

This product presents its maximum activity at basic solution pH, whereas at acid pH its activity is very low, and in particular its sporicidal power is practically non-existent.

On the other hand, when in solution at basic pH, glutaraldehyde has a very limited stability with time because it easily undergoes polycondensation with the formation of a polycondensate without activity. Compositions based on glutaraldehyde in aqueous solution have been formed comprising a buffer system in order to obtain solutions with a strictly controlled pH which is not excessively basic, for example between 7 and 7.4. Aqueous solutions of glutaraldehyde at acid pH which are stable and possess good bactericidal power have also been formed by adding non-ionic surface-active agents to the solution.

The present invention provides a new disinfectant and sterilising composition consisting of an aqueous solution of glutaraldehyde at pH 3.5—5 which in addition to glutaraldehyde also contains the following essential components:

a) an alkyl-, aryl- or halogen-substituted phenol or a thiobisphenol, in a quantity of 0.1—0.75 parts by weight per part by weight of glutaraldehyde;

b) a non-ionic surface-active agent of polyethoxylated type, in a quantity of 0.3—2.5 parts by weight per part by weight of glutaraldehyde.

The presence of a linear or branched alkanol of 2—5 carbon atoms is also preferred in a quantity of up to 10 parts by weight and in particular from 3 to 8 parts by weight per part by weight of glutaraldehyde.

Compositions of major practical interest are those which in the final aqueous solution give the following percentages of the various ingredients, but which must obviously be present in the aforesaid phenol/glutaraldehyde, surface-active agent/glutaraldehyde and alcohol/glutaraldehyde weight ratios:

| | |
|---|---|
| glutaraldehyde | 0.5—20% by weight |
| phenol | 0.1—10% by weight |
| surface-active agent | 1—30% by weight |
| alcohols | 1—20% by weight |

A particularly useful composition is the following:

| | |
|---|---|
| glutaraldehyde | 2% by weight |
| phenol | 1% by weight |
| surface-active agent | 3% by weight |
| alcohol | 16% by weight |

A particularly suitable alcohol for the composition of the present invention is isopropanol.

The non-ionic surface-active agent of polyethoxylated type is chosen from the following:

— alkylphenol with the alkyl of 8—9 carbon atoms, ethoxylated with 9—12 moles of ethylene oxide (EtO), and in particular ethoxylated nonylphenol;

— linear aliphatic alcohol of 11—16 carbon atoms, ethoxylated with 9—12 EtO.

The following phenols can be advantageously used in the compositions according to the invention:

o.phenylphenol

2,4,5-trichlorophenol

2,2'-dihydroxy-5,5'-dichloro-diphenylmethane (clorophene)

2,2'-dihydroxy-5,5'-dichloro-diphenylmonosulphide (fenticlor)

hexachloro-dihydroxy-diphenylmethane (hexachlorophene).

The concentration of the disinfectant sterilising composition according to the invention when in aqueous solution varies according to the particular type of disinfection or sterilisation for which it is to be used.

A concentration corresponding to 0.2% by weight of glutaraldehyde in the aqueous solution is suitable for the ambient-temperature sterilisation of diagnostic and surgical instruments in general within a time of 20 minutes. A concentration corresponding to 0.1% of glutaraldehyde is suitable for the ambient-temperature sterilisation of fibre optic equipment within a time of 10 minutes. A concentration of 0.06% is suitable for the aseptic preservation of instruments. The composition according to the invention can be prepared in the form of a high-concentration aqueous solution for dilution at the moment of use to the degree of dilution suitable for the specific application. Because of the high miscibility of the components with water, formulations can be prepared with a glutaraldehyde concentration of up to 20% by weight. The other ingredients are proportional to the glutaraldehyde present, in the aforesaid proportions. Compositions according to the invention in the form of a concentrated aqueous solution, and in particular containing 2% of glutaraldehyde by weight, are very stable with time, lasting at least 24 months.

They are also active in the presence of blood, proteins and organic materials. They can be advantageously used for sterilising and disinfecting the following instruments and articles:
— diagnostic apparatus
— endoscopy and urology apparatus (gastroscopes, cystoscopes, vascular and invasive probes)
— apparatus for anesthesia
— aerosol apparatus
— hemodialysis apparatus and microdiffusers
— dentistry apparatus
— plates for mesotherapy
— stainless steel, glass and rubber instruments.

An example of the preparation of the composition according to the invention is described hereinafter by way of illustration only.

Example
4 g of 50% glutaraldehyde are mixed with 20 ml of isopropanol and 30 g of distilled water. Separately, 1 g of o.phenylphenol, 3 g of nonylphenol polyethoxylated with 10 moles of EtO per mole, and 40 g of distilled water are mixed together at 40°C. The two solutions are mixed together and made up to 100 g with distilled water.

Bactericidal and sporicidal activity
The three following solutions were prepared, the first of which represents a composition according to the known art. The percentages are by weight.
A) 2% glutaraldehyde+0.25% non-ionic surface-active agent, in water
B) 2% glutaraldehyde, 1% o.phenylphenol, 3% non-ionic surface-active agent, in water
C) 2% glutaraldehyde, 1% o.phenylphenol, 3% non-ionic surface-active agent, 16% isopropanol.

Micro-organisms:
For our tests, 18 hour broth cultures of the following microorganisms forming part of our collection and originating from various pathological material were used.

As grams positive germs:
— S. aureus
— Streptococcus spp.
— Bacillus subtilis (and its spores)

As grams negative germs:
— Escherichia coli
— Klebsiella pneumoniae
— Serratia Marcescens
— Salmonella typhi
— Pseudomonas aeruginosa
— Proteus indole negative
— Proteus indole positive

As mycetes:
— Candida albicans
— Aspergillus spp.

Minimum inhibiting concentrations:
Broth cultures were prepared in Todd Hewitt broth (Difco) for the streptococci, in Mueller Hinton broth (Difco) for the other gram positive and gram negative bacterial strains, and in Sabouraud broth for the mycetes, and these were incubated at 37°C for 18 hours in the case of the bacteria and at 37°C for 48 or 72 hours in the case of the mycetes.

After suitable dilution ($10^{-3}$), a quantity of 0.1 ml of each dilution was inoculated into Todd Hewitt,

3

Mueller Hinton or Sabouraud broth respectively, containing dilutions on a doubling scale of the three solutions A, B and C being studied.

The inoculat consisted of $10^5$ CFUs (colony forming units).

After incubation at 37°C for 18 hours or for 72 hours for the mycetes, the MIC (minimum inhibiting concentration) was determined as the lowest antibiotic concentration able to completely inhibit the growth of the germ under examination.

Minimum bactericidal concentrations:

Subcultures on suitable growth culture media to which agar had been added were prepared from the tubes containing the minimum inhibiting disinfectant concentrations and from the two tubes containing the two greater disinfectant concentrations on the doubling scale.

These inoculated culture media were then incubated at the appropriate temperatures for the required times.

The reading was taken in CFUs (colony forming units).

The minimum bactericidal concentration was considered to be that disinfectant concentration for which the subculture was sterile.

Determination of contact times:

Subcultures of four chosen test micro-organisms were prepared at determined time intervals from concentrations on a doubling scale of the three solutions under examination.

The chosen micro-organisms were:
— S. aureus
— B. subtilis
— P. aeruginosa
— C. albicans.

The chosen time intervals were 30 seconds, 1, 2, 5, 10, 20 and 30 minutes from the preparation of the starting culture.

After preparing these subcultures the inoculated tubes were incubated at 37°C for the appropriate times.

The reading was taken by checking the turbidity attributable to any growth which had taken place.

Conclusive results and considerations:
— Minimum inhibiting concentrations

The values obtained as minimum inhibiting concentrations of the tested micro-organisms are given in the accompanying Tables 1, 2 and 3.

Good synergic action of the three active principles examined can be deduced from these. In this respect, the values obtained improved continuously in passing from solution A to solution B and then to solution C.

Whereas the MIC values for solutions A and B are approximately 1/4 and 1/8, a considerably lower MIC is obtained for solution C, namely down to 1/64 of the mother solution.

— Minimum bactericidal concentrations

All the MIC values obtained for the germs chosen for the test can be superimposed on the MBC values. This ensures rapid bactericidal, fungicidal and sporicidal activity of the solutions, as can be seen from Table 4.

— Contact times

The values given as contact times necessary for killing the tested micro-organisms are shown in the accompanying Tables 5, 6, 7 and 8.

The contact times obtained decrease in progressing through the three respective solutions.

TABLE 1

Minimum inhibiting concentrations for gram positive germs

| Micro-organism | Solution A | Solution B | Solution C |
|---|---|---|---|
| S. aureus 1 | 1/4 | 1/8 | 1/16 |
| S. aureus 2 | 1/4 | 1/4 | 1/16 |
| S. aureus 3 | 1/2 | 1/4 | 1/16 |
| Streptococcus 1 | 1/4 | 1/8 | 1/32 |
| Streptococcus 2 | 1/8 | 1/16 | 1/32 |

4

TABLE 2
Minimum inhibiting concentrations for gram-negative germs

| Micro-organism | Solution A | Solution B | Solution C |
|---|---|---|---|
| Escherichia coli | 1/2 | 1/4 | 1/8 |
| Klebsiella pneumoniae | 1/4 | 1/8 | 1/32 |
| S. marcescens | 1/2 | 1/4 | 1/32 |
| S. typhi | 1/2 | 1/4 | 1/32 |
| P. aeruginosa | 1/4 | 1/8 | 1/64 |
| P. ind+ | 1/2 | 1/8 | 1/64 |
| P. ind− | 1/4 | 1/8 | 1/32 |

TABLE 3
Minimum inhibiting concentrations for sporogenic germs

| Micro-organism | Solution A | Solution B | Solution C |
|---|---|---|---|
| B. subtilis 1 | 1/4 | 1/8 | 1/16 |
| B. subtilis 2 | 1/8 | 1/16 | 1/64 |
| B. subtilis 3 | 1/8 | 1/32 | 1/64 |
| Spores | 1/8 | 1/8 | 1/32 |
| | 1/2 | 1/2 | 1/16 |

TABLE 4
Minimum concentrations for mycetes

| Micro-organism | Solution A | Solution B | Solution C |
|---|---|---|---|
| C. albicans 1 | 1/4 | 1/8 | 1/16 |
| C. albicans 2 | 1/2 | 1/4 | 1/16 |
| C. albicans 3 | 1/4 | 1/16 | 1/64 |

TABLE 5
Contact times for gram positive germs

| Micro-organism | Solution A | Solution B | Solution C |
|---|---|---|---|
| S. aureus 1 | 2 min | 1 min | 30 sec |
| S. aureus 2 | 5 min | 2 min | 30 sec |
| S. aureus 3 | 10 min | 5 min | 1 min |
| Streptococcus 1 | 2 min | 2 min | 30 sec |
| Streptococcus 2 | 2 min | 1 min | 30 sec |

### TABLE 6
Contact times for gram negative germs

| Micro-organism | Solution A | Solution B | Solution C |
|---|---|---|---|
| Escherichia coli | 5 min | 2 min | 1 min |
| Klebsiella pneum. | 10 min | 5 min | 2 min |
| S. marcescens | 10 min | 2 min | 1 min |
| S. typhi | 10 min | 2 min | 2 min |
| P. aeruginosa | 5 min | 2 min | 30 sec |
| P. ind.+ | 2 min | 1 min | 30 sec |
| P. ind.− | 2 min | 30 sec | 30 sec |

### TABLE 7
Contact times for sporogenic germs and their spores

| Micro-organism | Solution A | Solution B | Solution C |
|---|---|---|---|
| B. subtilis 1 | 10 min | 5 min | 2 min |
| B. subtilis 2 | 5 min | 1 min | 30 sec |
| B. subtilis 3 | 5 min | 2 min | 30 sec |
| Spores | 20 min | 10 min | 5 min |
| | 30 min | 10 min | 2 min |
| | 30 min | 20 min | 10 min |

### TABLE 8
Contact times for mycetes

| Micro-organism | Solution A | Solution B | Solution C |
|---|---|---|---|
| C. albicans 1 | 20 min | 20 min | 10 min |
| C. albicans 2 | 10 min | 10 min | 2 min |
| C. albicans 3 | 30 min | 10 min | 5 min |

**Claims for the Contracting States: BE, CH, DE, FR, GB, IT, LI, LU, NL, SE**

1. A disinfectant and sterilising composition consisting of an aqueous solution of glutaraldehyde of pH 3.5—5 containing in addition to glutaraldehyde the following essential components:

a) an alkyl-, aryl-, or halogen-substituted phenol or a thiobisphenol, in a quantity of 0.1—0.75 parts by weight per part by weight of glutaraldehyde;

b) a non-ionic surface-active agent of polyethoxylated type, in a quantity of 0.3—2.5 parts by weight per part by weight of glutaraldehyde, and chosen from:

— alkylphenol with the alkyl of 8—9 C atoms, ethoxylated with 9—12 moles of ethylene oxide

— linear aliphatic alcohol of 11—16 C atoms, ethoxylated with 9—12 moles of ethylene oxide.

2. A composition as claimed in claim 1, also containing a linear or branched alkanol of 2—5 C atoms in a quantity of up to 10 parts by weight, and preferably from 3 to 8 parts by weight, per part by weight of glutaraldehyde.

3. A composition as claimed in claim 1, containing from 0.5 to 20% of glutaraldehyde by weight.

4. A composition as claimed in claim 2, containing:

| | |
|---|---|
| glutaraldehyde | 2% by weight |
| phenol | 1% by weight |
| non-ionic surface-active agent | 3% by weight |
| alcohol | 16% by weight |

5. A composition as claimed in claim 1, wherein the phenol is chosen from:

o.phenylphenol
2,4,5-trichlorophenol
2,2'-dihydroxy-5,5'-dichloro-diphenylmethane
2,2'-dihydroxy-5,5'-dichloro-diphenylmonosulphide
2,2'-dihydroxy-3,3',5,5',6,6'-hexachloro-diphenylmethane.

**Claims for the Contracting States: AT, GR, SP**

1. A process for disinfecting and sterilizing environments, equipments and devices used in diagnostic and therapeutic field and of clothes of the patients and of the assisting personnel, consisting in the treatment of said objects with an aqueous solution of glutaraldehyde of pH 3.5—5 containing in addition to glutaraldehyde the following essential components:
   a) an alkyl-, aryl-, or halogen-substituted phenol or a thiobisphenol, in a quantity of 0.1—0.75 parts by weight per part by weight of glutaraldehyde;
   b) a non-ionic surface-active agent of polyethoxylated type, in a quantity of 0.3—2.5 parts by weight per part by weight of glutaraldehyde, and chosen from:
   — alkylphenol with the alkyl of 8—9 C atoms, ethoxylated with 9—12 moles of ethylene oxide;
   — linear aliphatic alcohol of 11—16 C atoms, ethoxylated with 9—12 moles of ethylene oxide.

2. A process according to claim 1, wherein an aqueous solution of glutaraldehyde is used which also contains a linear or branched alkanol of 2—5 C atoms in a quantity of up to 10 parts by weight, and preferably from 3 to 8 parts by weight, per part by weight of glutaraldehyde.

3. A process according to claim 1, wherein an aqueous solution of glutaraldehyde is used which contains a substituted phenol chosen from:
   o.phenylphenol
   2,4,5-trichlorophenol
   2,2'-dihydroxy-5,5'-dichloro-diphenylmethane
   2,2'-dihydroxy-5,5'-dichloro-diphenylmonosulphide
   2,2'-dihydroxy-3,3',5,5',6,6'-hexachloro-diphenylmethane.

**Patentansprüche für den Vertragsstaaten: BE, CH, DE, FR, GB, IT, LI, LU, NL, SE**

1. Desinfektions- und Sterilisationsmittel das aus einer wäßrigen Lösung von Glutaraldehyde (pH 3.5—5) besteht die außer Glutaraldehyde auch die folgenden Substanzen enthält:
   a) Alkyl-, Aryl- oder Halogensubstituiertes Phenol oder Thiobisphenol, in einer Menge von 0,1—0,75 Gew.-Teile je Gew.-Teile von Glutaraldehyde
   b) eine nicht-ionische Oberfläche-aktive Substanz des polyethoxylierten Typ, in einer Menge von 0.3—2.5 Gew.-Teile je Gew.-Teile von Glutaraldehyde und ausgewählt zwischen:
   — Alkylphenol, wo die Alkylgruppe ein $C_{8-9}$ Alkyl ist, ethoxyliert mit 9—12 Molen von Ethyleneoxide
   — Geradkettiges aliphatisches Alkohol mit $C_{11}$—$C_{16}$, ethoxyliert mit 9—12 Molen von Ethyleneoxide.

2. Desinfektions- und Sterilisationsmittel gemäß Anspruch 1 das auch ein geradkettiges oder verzweigtes Alkanol $C_{2-5}$ in einer Menge bis zu 10 Gew.-Teile, vorzugsweise von 3 bis zu 8 Gew.-Teile, je Gew.-Teile von Glutaraldehyde enthält.

3. Desinfektions- und Sterilisationsmittel gemäß Anspruch 1 das von 0.5 bis 20% Gew. von Glutaraldehyde enthält.

4. Desinfektions- und Sterilisationsmittel gemäß Anspruch 2 das

| | |
|---|---|
| Glutaraldehyde | 2% Gew. |
| Phenol | 1% Gew. |
| Nicht-ionische Oberfläche-aktive Substanz | 3% Gew. |
| Alkohol | 16% Gew. |

enthält.

5. Desinfektions- und Sterilisationsmittel gemäß Anspruch 1 wobei das Phenol zwischen:
   O-Phenol
   2,4,5-Trichlorophenol

7

# EP 0 255 875 B1

2,2′-Dihydroxy-5,5′-dichloro-diphenylmethane
2,2′-Dihydroxy-5,5′-dichloro-diphenylmonosulphide
2,2′-dihydroxy-3,3′,5,5′,6,6′-hexachloro-diphenylmethane ausgewählt wird.

**Patentansprüche für die Vertragsstaaten: AT, GR, SP**

1. Verfahren zur Desinfektion und Sterilisation von Räumen, Geräten und Gegenständen die in die diagnostische und terapeutische Gebiete benutzt werden und von Kleidungen der Patienten und der Gehilfer, wobei die obengenannten Geräte mit einer wäßrigen Lösung von Glutaraldehyde (pH 3.5—5) die außer Glutaraldehyde auch die folgenden Substanzen enthält:

a) Alkyl-, Aryl- oder Halogensubstituiertes Phenol oder Thiobisphenol, in einer Menge von 0,1—0,75 Gew.-Teile je Gew.-Teile von Glutaraldehyde

b) eine nicht-ionische Oberfläche-aktive Substanz des polyethoxylierten Typ, in einer Menge von 0,3—2,5 Gew.-Teile je Gew.-Teile von Glutaraldehyde und ausgewählt zwischen:

— Ein Alkylphenol, wo die Alkylgruppe ein $C_{8-9}$ Alkyl ist, ethoxyliert mit 9—12 Molen von Ethyleneoxide

— Ein Geradkettiges aliphatisches Alkohol mit $C_{11-16}$, ethoxyliert mit 9—12 Molen von Ethyleneoxide behandelt werden.

2. Verfahren gemäß Anspruch 1 wobei eine wäßrige Lösung die auch ein geradkettiges oder verzweigtes Alkanol $C_{2-5}$ in einer Menge von bis zu 10 Gew.-Teile, vorzugsweise von 3 bis zu 8 Gew.-Teile, je Gew.-Teile von Glutaraldehyde enthält, benutzt wird.

3. Verfahren gemäß Anspruch 1 wobei eine wäßrige Lösung von Glutaraldehyde die ein substituiertes Phenol ausgewählt zwischen:

O-Phenol

2,4,5-Trichlorophenol

2,2′-Dihydroxy-5,5′-dichloro-diphenylmethane

2,2′-Dihydroxy-5,5′-dichloro-diphenylmonosulphide

2,2′-dihydroxy-3,3′,5,5′,6,6′-hexachloro-diphenylmethane
enthält, benutzt wird.

**Revendications pour les Etats Contractants: BE, CH, DE, FR, GB, IT, LI, LU, NL, SE**

1. Composition pour désinfecter et stériliser constituée par une solution aqueuse de glutaraldéhyde (pH 3.5—5) et contenante en plus de la glutaraldéhyde les composés suivants:

a) un phénol substituè par alkyl, aryl ou halogèn ou un thiobisphénol, dans une quantité de 0.1—0.75 parties en poids par partie en poids de glutaraldéhyde;

b) un agent non-ionique ayant activité superficielle du type des composés polyethoxylés, dans une quantité de 0.3—2.5 parties en poids par partie en poids de glutaraldéhyde, et choisi parmi:

— alkylphénol avec le groupe alkyl $C_{8-9}$, ethoxylé avec 9

— 12 moles de éthylèneoxide

— alcool aliphatique linéaire $C_{11-16}$ ethoxylé avec 9—12 moles de éthylèneoxide.

2. Composition selon la revendication 1 contenante aussi un alcanol linéaire ou ramifié $C_{2-5}$ dans une quantité jusqu'à 10 parties en poids, et préférablement de 3 a 8 parties en poids par partie en poids de glutaraldéhyde.

3. Composition selon la revendication 1, contenante 0.5—20% de glutaraldéhyde en poids.

4. Composition selon la revendication 2 contenante:

| | |
|---|---|
| glutaraldéhyde | 2% en poids |
| phénol | 1% en poids |
| Agent non ionique ayant activité superficielle | 3% en poids |
| alcool | 16% en poids |

5. Composition selon la revendication 1, où le phénol est choisi parmi:

o-phénol

2,4,5-trichlorophenol

2,2′-dihydroxy-5,5′-dichloro-diphénylmethane

2,2′-dihydroxy-5,5′-dichloro-diphénylmonosulphide

2,2′-dihydroxy-3,3′,5,5′,6,6′-hexachloro-diphénylmethane

**Revendications pour les Etats Contractants: AT, GR, SP**

1. Procédé pour la désinfection et stérilisation des ambiants, équipement et appareils employès dans le domaine diagnostique et thérapeutique comme pour les vêtements des patients et du personnel assistant où les objects sont traités avec une solution aqueuse de glutaraldéhyde (pH 3.5—5) et contenante en plus de la glutaraldéhyde les composés suivants:

8

a) un phénol substituè alkyl, aryl ou halogèn ou un thiobisphénol, dans une quantité de 0.1—0.75 parties en poids par partie en poids de glutaraldéhyde;

b) un agent non-ionique ayant activité superficielle du type des composés polyéthoxylés, dans une quantité de 0.3—2.5 parties en poids par partie en poids de glutaraldéhyde, et choisi parmi:
— alkylphénol avec le groupe alkyl $C_{8-9}$, éthoxylé avec 9—12 moles de éthylèneoxide
— alcool aliphatique linéaire $C_{11-16}$ éthoxylé avec 9—12 moles de éthylèneoxide.

2. Procédé selon la revendication 1 où on emploie une solution aqueuse contenante aussi un alcanol linéaire ou ramifié $C_{2-5}$ dans une quantité jusqu'à 10 parties en poids, et préférablement de 3 a 8 parties en poids par partie en poids de glutaraldéhyde.

3. Procédé selon la revendication 1, où l'on emploie une solution aqueuse de glutaraldéhyde contenente un phénol substituè choisi parmi:

o-phénol

2,4,5-trichlorophénol

2,2'-dihydroxy-5,5'-dichloro-diphénylmethane

2,2'-dihydroxy-5,5'-dichloro-diphénylmonosulphide

2,2'-dihydroxy-3,3',5,5',6,6'-hexachloro-diphénylmethane.